# EUROPEAN PATENT APPLICATION

(11) **EP 1 967 193 A1**
(43) Date of publication of application: **10.09.2008**
(21) Application number: 07103530.7
(22) Date of filing: 05.03.2007
(51) Int. Cl.: A61K 31/704, A61K 31/7048, A61P 35/00

(54) **Enhancement of anticancer therapy by flavonoids**

(71) Applicant: Vrije Universiteit Medisch Centrum (VUMC), 1081 HV Amsterdam (NL)
(72) Inventor: The designation of the inventor has not yet been filed
(74) Representative: van Loon, C.J.J.

(57) **Abstract**

The present invention provides means and methods for the treatment of an individual suffering from cancer. A group of polyphenolic flavonoids where found to have synergistic anti-tumor activity with a chemotherapeutic drug.

## Description

The invention relates to the synergistic use of a polyphenolic flavonoid and a chemotherapeutic drug for treating an individual suffering from cancer.

Cancer is the uncontrolled growth and spread of cells that may affect almost any tissue of the body. Lung, colorectal and stomach cancer are among the five most common cancers in the world for both men and women. Among men, lung and stomach cancer are the most common cancers worldwide. For women, the most common cancers are breast and cervical cancer. More than 11 million people are yearly diagnosed with cancer worldwide, of which more than 60% will die because of the cancer.

Conventional treatment comprises surgery, radiation, chemotherapy, hormonal therapy and immunotherapy. Hormone therapy, chemotherapy and immunotherapy are systemic treatments which may affect cancer cells throughout the body, while surgery and radiation are used to eliminate or eradicate local cancer cells.

Surgery involves the removal of the cancer and some tissue adjacent to the cancer. Radiation or radiotherapy, uses high-energy rays to damage or kill cancer cells by preventing them from growing and dividing. Chemotherapy involves the use of drugs, often termed chemotherapeutic drugs, to eliminate cancer cells. Chemotherapy is often used alone, or in conjunction with radiation therapy or surgery. Hormonal therapy is often used to eliminate cancer cells that are responsive to hormones such as oestrogen and androgen. Typical examples of such cancer cells are breast cells and prostate cells. Hormonal therapy includes the use of drugs that block hormone production, the removal of organs that secrete hormones, and the use of drugs that change the way hormones work, such as anti-hormones. Other systemic anticancer therapies are immunotherapy and targeted therapy. Immunotherapy comprises the use of interferon and other cytokines to stimulate the natural defense system to eliminate cancer cells, and also includes the use of foreign antibodies and cancer vaccines.

Conventional cancer treatments, such as chemotherapy and radiation therapy, do not distinguish between cancer cells and healthy cells. Chemotherapy aims at eliminating rapidly dividing cells in general, which is a hallmark of cancer cells. However, there are also healthy cells that can be rapidly dividing, such as blood cells and the cells lining the mouth and the gut, and which therefore may also be damaged by chemotherapy. Similarly, radiation therapy kills healthy cells that are in the path of the radiation. The damage of healthy cells may result in side effects, such as nausea, vomiting, hair loss and mouth sores. These side effects may be severe, and thereby reducing the quality of life of a particular patient. In some cases, the side effects may even prevent full treatment of a particular patient. However, many side effects can be prevented or controlled, allowing many people to normally take part in life while receiving chemotherapy.

For example, the administration of doxorubicin to patients is limited by a dose-dependent cardiotoxicity, which can lead to late side-effects resulting in severe morbidity and also mortality. Combining doxorubicin treatment with other anticancer drugs, e.g. taxanes, increases efficacy, but unfortunately also augments cardiotoxicity (Minotti et al., 2004. Pharmacol Rev 56: 185; Seidman et al., 2002. J Clin Oncol 20: 1215). Preclinical experiments showed that a polyphenolic flavonoid such as 7-monohydroxyethylrutoside (monoHER) is a potential protective agent against doxorubicin-induced cardiotoxicity that has no interference with its antitumor activity (Van Acker SA et al., 1997. Clin Cancer Res 3: 1747; Van Acker et al., 2000. Clin Cancer Res 6: 1337). The radical scavenging and iron chelation properties of monoHER are supposed to be the mechanisms of action (Haenen et al., 1993. Venoruton. Phlebology : S10; Van Acker et al., 1993. Phlebology: S31). In a phase I study the possible side effects and pharmacokinetics of monoHER were evaluated. No side-effects were observed and the potentially cardioprotecting pharmacokinetic values for Cmax and AUC^{∞} were achieved (Willems et al., 2006. Cancer Chemother Pharmacol 57:678).

Many patients diagnosed with cancer receive chemotherapy. For cancers that respond well to the prescribed chemotherapeutic drugs, this approach will treat the cancer effectively. However, several cancers do not respond, or only marginally respond, to the prescribed chemotherapeutic drugs. Therefore, it is an object of the present invention to enhance the chemotherapeutic effect of a chemotherapeutic drug in humans.

Therefore, in one aspect the invention provides the use of a polyphenolic flavonoid in the manufacture of a medicament for the treatment of an individual suffering from cancer, whereby said flavonoid acts synergistically with a chemotherapeutic drug; and whereby said individual is a human.

Upon conducting phase II experiments, the inventors surprisingly established that a polyphenolic flavonoid such as monoHER, a compound that was thusfar used to prevent cardiotoxicity induced by a chemotherapeutic drug, synergistically increases efficacy of said chemotherapeutic drug.

A chemotherapeutic drug is preferably a drug that affects rapidly dividing cells such as cancer cells. In general, such a chemotherapeutic drug acts by hampering or preventing proliferation of cells by damaging the DNA, inhibiting the synthesis or replication of DNA, or blocking the separation of the daughter cells. Commonly used chemotherapeutic drugs can be grouped into alkylating agents such as cyclophosphamide and cisplatin, nitrosoureas such as carmustine and semustine, antimetabolites such as mercaptopurine and 5-fluorouracil, antibiotics such as anthracyclines and mitomycin-C, alkaloids such as vincristine and taxane, and hormones such as tamoxifen.

In a preferred embodiment, said chemotherapeutic drug comprises an anthracycline.

Anti-tumor antibiotics in general are known to eliminate cancer cells by preventing DNA replication and/or RNA synthesis. Anthracyclines have been reported to inhibit proliferation of cancer cells by multiple mechanisms, including but not limited to intercalating with cellular DNA, binding to cell membranes, altering ion transport, and generating oxygen radicals that induce DNA breaks. Anthracyclines, such as daunorubicin, idarubicin, doxorubicin, and epirubicin, are used to treat multiple cancer types, including breast cancer, ovarian cancer, soft tissue and osteogenic sarcomas, neuroblastoma, lymphoma, and leukemia.

The anthracycline doxorubicin (DOX) is widely used in the treatment of several solid tumors and hematological malignancies in adult and pediatric patients. Treatment with DOX is limited by a dose-dependent cardiotoxicity, which may lead to late side effects resulting in severe morbidity and mortality (1, 2). Although the 5-year survival of childhood cancer has improved from 30% to 70% in the last 40 years, the risk of death from cardiac events in these survivors is eight times higher than that in the normal population (3). Besides this, combining DOX with other anticancer drugs, e.g. taxanes and trastuzumab, increases efficacy, but unfortunately also augments cardiotoxicity (4, 5).

Although the mechanism of DOX-induced cardiotoxicity is still not fully understood, a major role has been ascribed to the induction of free radicals (6-8). The cardiomyocyte is particularly vulnerable to free radical injury because of properties such as a low antioxidant status (9, 10).

Presently, the cardioprotectant dexrazoxane is the only drug with proven efficacy (11). A recent review recommended the use of dexrazoxane if the risk of cardiotoxicity is high. However, clinicians should weigh the cardioprotective effect of this agent against the risk of a potential decrease of antitumor activity for the individual patient (12).

Preclinical experiments showed that the flavonoid 7-monohydroxyethylrutoside (monoHER) is also a potential protective agent against DOX-induced cardiotoxicity without interfering with its antitumor activity (13, 14). The radical scavenging and iron chelating properties of monoHER are its supposed mechanisms of action (15, 16). No serious side effects were observed in a clinical phase I study up to a dose of 1500 mg/m². At this dose the pharmacokinetic end-points were reached, i.e. Cmax and AUC were comparable to those obtained in mice under protecting conditions. Therefore this dose was considered feasible and safe to be evaluated in a phase II study (17). In the present invention it is preferred that the chemotherapeutic drug is doxorubicin.

In a preferred embodiment, said polyphenolic flavonoid comprises a compound according to formula 1: 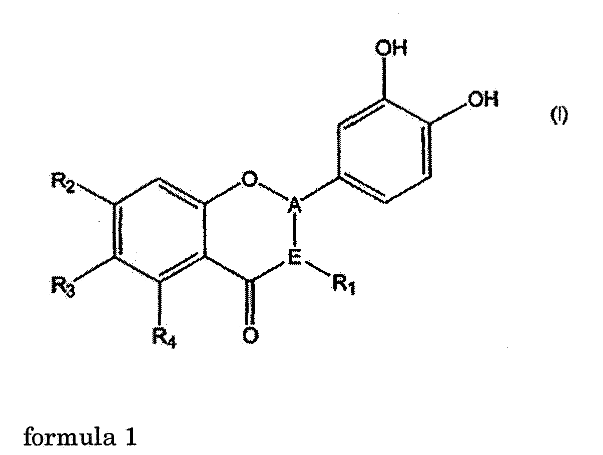 wherein:
A and E form together a C-C or C=C bond,
R₁, R₂, R₃, and R₄ are each independently chosen from (or selected from the group consisting of):
   H;
   OH;
   (R')ₙ R" and/or O(R')ₙ R" wherein n=0-8 and
      R' is independently chosen from CH₂ or O(CH₂)_{n'} wherein n'=1-4 and
      R" when
         n=0 is independently chosen from
            H;
            an aromatic group; a C₁-C₈ alkyl group; a sugar in mono, di or trimeric form or an analogue thereof;
            halide;
            NHR'''; N(R''')₂; N(R''')₃-halicie;
            COOR''';
            CONHR'''; CON(R''')₂; or CON(R''')₃-halide wherein
               R''' is independently chosen from H; an aromatic group; a C₁-C₈ alkyl group;or a sugar in mono, di or trimeric form or an analogue thereof;
            and when
         n=1-8 is independently chosen from
            H;
            an aromatic group; a C₁-C₈ alkyl group; a sugar in mono, di or trimeric form or an analogue thereof; NHR'''; N(R''')₂; N(R''')₃-halide;;
            OH;
            halide;
            COOR''';
            CONHR'''; CON(R''')₂; or CON(R''')₃-halide wherein
               R''' is independently chosen from H; an aromatic group; a C₁-C₈ alkyl group; or a sugar in mono, di or trimeric form or an analogue thereof;
   (R')ₙ OR" wherein n=1-8 and
      R' is independently chosen from CH₂ or O(CH₂)_{n'} wherein n'=1-4 and
      R" is independently chosen from
         H;
         an aromatic group; a C₁-C₈ alkyl group; a sugar in mono, di or trimeric form or an analogue thereof;
         NHR'''; N(R''')_{2;} N(R''')₃-halide;
         H;
         halide;
         COOR''';
         CONHR'''; CON(R''')₂ or CON(R''')₃-halide wherein
         R''' is independently chosen from H; an aromatic group; a C₁-C₈ alkyl group; or a sugar in mono, di or trimeric form or an analogue thereof;
wherein n, n' and q are integers and wherein the number range indication letter=(number 1)-(number 2) means that the letter can be any of the integers from and including number 1 to and including number 2. The alternatives for each R₁; R_{2;} R_{3;} R_{4;} R';R" and R''' can be independently chosen.

Polyphenolic flavonoids comprise a class of naturally occurring benzo-γ-pyron derivatives that are ubiquitous in photosynthesizing cells and provide colour, flavour, anti-fungal and antibacterial activity. Sources of natural flavonoids comprise dark chocolate, strawberries, blueberries, cinnamon, pecans, walnuts, grapes, lemons, and cabbage. Besides their relevance in plants, they are pharmacologically important as they comprise iron-chelating and radical-scavenging properties, which may contribute to the antioxidant activity of flavonoids. Fully synthetic and semi-synthetic compounds have recently become available.

Natural polyphenolic flavonoids comprise flavon(ol)es such as quercitin and rutin, flavonon(ol)es such as naringin and hesperetin, flavones such as luteolin, flavanoles such as catechin, chalcones such as phloretin, anthocyanidins such as cyanidin and isoflavones such as genistein.

In yet a further preferred embodiment, said polyphenolic flavonoid comprises a compound according to formula 1 (*supra*) with the proviso that said compound is not a compound wherein R₁= OH, R₂= OH; R₃=H and R₄=OH and A and E together form a C=C bond. Preferably said compound is not quercitin.

Semi-synthetic polyphenolic flavonoids comprise hydroxyethylated-derivatives such as hydroxyethylrutosides among which 7-monohydroxyethylrutoside (mono-HER). Voorkeur voor MonoHer In a further preferred embodiment, said polyphenolic flavonoid comprises monohydroxyethylrutoside.

Synthetic polyphenolic flavonoids, and novel methods for their production, have been described in WO/2001/21608, which is incorporated herein by reference.

Semisynthetic and synthetic polyphenolic flavonoids have advantages over natural polyphenolic flavonoids. It was surprisingly found by the present inventors that the semisynthetic polyphenolic flavonoid monoHER and compounds of formula 1 that have the same or similar R₁, R₂, R₃ or R₄ groups are with respect to their anti-tumor activity more synergistic with a chemotherapeutic drug. Thus preferably said polyphenolic flavonoid of formula 1 is a synthetic or semi-synthetic flavonoid. Preferably said flavonoid of formula 1 is not a naturally occurring flavonoid. In a preferred embodiment said compound of formula I is a hydroxy-ethylated or hydroxy-methylated flavonoid. In a particularly preferred embodiment said polyphenolic flavonoid comprises monoHER.

Chemotherapeutic drugs are used for the treatment of a diverse set of cancer types. Cancers can be classified either according to the kind of fluid or tissue from which they originate, or according to the location in the body where they first developed. Cancer types that might be treated by a synergistic effect of a polyphenolic flavonoid and a chemotherapeutic drug according to the invention include, but are not limited to, a carcinoma, which is a cancer of epithelial tissue that covers or lines surfaces of organs, glands, or body structures and which account for 80 percent to 90 percent of all cancer cases; a sarcoma, which is a cancer growing from connective tissues, such as cartilage, fat, muscle, tendons, and bones; a lymphoma, which is a cancer type that originates in the nodes or glands of the lymphatic system; a leukemia, which is a cancer of the bone marrow; or a myeloma, which is a cancer that grows in the plasma cells of bone marrow.

In this aspect, the invention preferably provides the use of a polyphenolic flavonoid in the manufacture of a medicament for the treatment of an individual suffering from cancer, wherein said cancer comprises a soft tissue sarcoma.

The inventors have surprisingly found that a polyphenolic flavonoid acts synergistically with a chemotherapeutic drug for the treatment of soft tissue sarcomas.

The term soft tissue refers to tissues that connect, support, or surround other structures and organs of the body. Soft tissue includes muscles, tendons, fibrous tissues, fat, blood vessels, nerves, and synovial tissues. Soft tissue sarcomas in general share clinical and microscopic characteristics that are different from osteosarcomas and are treated differently from said osteosarcomas.

Some tumors of the soft tissue are termed benign or non-cancerous. These tumors do not spread and are rarely life-threatening. However, some benign tumors can cause symptoms due to interference with normal body functions.

Sarcomas can invade surrounding tissue and can metastasize to other organs of the body, forming metastases. The cells of these metastases or secondary tumors are similar to those of the primary cancer.

Therefore, the invention further provides the use of a polyphenolic flavonoid in the manufacture of a medicament for the treatment of an individual suffering from a sarcoma, comprising the treatment of an individual suffering from metastases of said sarcoma.

In a preferred embodiment, said sarcoma is selected from the group comprising alveolar soft part sarcoma, angiosarcoma, dermatofibrosarcoma, desmoid tumor, desmoplastic small round cell tumor, mesenchymoma, clear cell sarcoma, chondrosarcoma, fibrosarcoma, hemangiopericytoma, hemangiosarcoma, Kaposi's sarcoma, leiomyosarcoma, liposarcoma, lymphangiosarcoma, malignant fibrous histiocytoma, neurofibrosarcoma, rhabdomyosarcoma , synovial sarcoma,and peripheral nerve sheet tumor.

In yet a further preferred embodiment, said sarcoma is selected from the group comprising fibrous histiocytoma and peripheral nerve sheet tumor. Polyphenolic flavonoids have been used in preclinical experiments to test their cardioprotective effects upon treatment of the animals with an anthracycline such as doxorubicin (van Acker et al., Clin Cancer Res. 1997. 3(10):1747). From a first set of experiments, it was concluded that polyphenolic flavonoids such as monoHER showed a dose-dependent cardioprotection against doxorubicin-induced chronic cardiotoxicity and did not influence the antitumor activity of doxorubicin in vitro or in vivo. However, subsequent experiments indicated that this cardioprotective effect was not prolonged during a half year observation period, suggesting that the dose and frequency of administration of polyphenolic flavonoids have an effect.

Polyphenolic flavonoids can be administered into the body by several routes. They may be taken orally, injected intravenously, intramuscularly, intrathecally, or subcutaneously, or introduced by insertion into the rectum or vagina. When injected intravenously, said polyphenolic flavonoid is immediately delivered to the bloodstream and tends to take effect more quickly than when given by any other route.

Polyphenolic flavonoids can also be given by intravenous, intramuscular, intrathecal, or subcutaneous administration. Parameters that are preferably controlled during administration of the polyphenolic flavonoid are dose and time.

Therefore, in another embodiment, said polyphenolic flavonoid is administered to said individual by intravenous infusion at a dose of between 100 and 3000 mg/m².

In a preferred embodiment, said polyphenolic flavonoid is administered to said individual by intravenous infusion at a dose of 1500 mg/m².

In a further preferred embodiment, said polyphenolic flavonoid is administered to said individual by infusion of a total volume of between about 1 and about 1000 ml, more preferred between about 2 and about 500 ml, more preferred between about 5 and about 250 ml. The limitations provided may prevent fractional changes in blood volume and may prevent further complications.

A polyphenolic flavonoid of the invention can be administered to the individual during the administration of said chemotherapeutic drug, after administering said chemotherapeutic drug, or prior to administering said chemotherapeutic drug. If the polyphenolic flavonoid is administered simultaneously with said chemotherapeutic drug, said polyphenolic flavonoid and said chemotherapeutic drug may be comprised in one pharmaceutical composition, or in more than one pharmaceutical composition, further comprising an acceptable carrier. A polyphenolic flavonoid of the invention is preferably administered prior to administration of the chemotherapeutic drug.

It is possible to administer the polyphenolic flavonoid or the chemotherapeutic drug in a very short time for instance via intravenous injection or over an extended period of time. Preferably, however the polyphenolic flavonoid or the chemotherapeutic drug is administered over a time period between 0 and 24 hours, preferably over a period of 0-12 hours and more preferably over a period of 0-6 hours. A practical time frame is between 0 and 2 hours.

While it is possible to administer the polyphenolic flavonoid and the chemotherapeutic drug simultaneously, it is preferred that the individual is first provided with the polyphenolic flavonoid and subsequently with the chemotherapeutic drug. In this embodiment it is possible to start administration of the chemotherapeutic drug before completion of the administration of said polyphenolic flavonoid of the invention. In another embodiment it is preferred that the polyphenolic flavonoid administration is completed before the administration of the chemotherapeutic drug is started. In a preferred embodiment, the time interval between the end of administration of the polyphenolic flavonoid and the start of administration of the chemotherapeutic drug is between 0 and 24 hours, more preferably between 0 and 12 hours, more preferred between 0 and 6 hours, even more preferred between 0 and 2 hours.

When the chemotherapeutic drug administration is started prior to the start of administration of the polyphenolic flavonoid of the invention, it is preferred that polyphenolic flavonoid administration is started as early as possible after initiation of the administration of said chemotherapeutic drug. Preferably said polyphenolic flavonoid administration is started between 0-24 hours of initiation of the administration of said chemotherapeutic drug. More preferred between 0-12, more preferred between 0-6 and even more preferred between 0-2 hours of initiation of the administration of said chemotherapeutic drug.

In yet another aspect, the invention provides a method for treating an individual suffering from cancer, comprising administering to said individual a medicament comprising a polyphenolic flavonoid and a chemotherapeutic drug, whereby said flavonoid acts synergistically with said chemotherapeutic drug; and whereby said individual is a human.

In one embodiment, said chemotherapeutic drug comprises an anthracycline, preferably said anthracycline is doxorubicin.

In a further embodiment, said polyphenolic flavonoid comprises a compound according to formula 1 (*supra*).

In yet a further embodiment, the invention provides a method for treating an individual suffering from cancer, comprising administering to said individual a medicament comprising a polyphenolic flavonoid and a medicament comprising a chemotherapeutic drug, wherein said medicament comprising a polyphenolic flavonoid is administered prior to administering said medicament comprising a chemotherapeutic drug.

In another aspect, the invention provides the use of a polyphenolic flavonoid for the manufacture of a medicament to enhance the chemotherapeutic effect of a chemotherapeutic drug in human.

In yet another aspect, the invention provides a pharmaceutical composition, comprising a polyphenolic flavonoid and an acceptable carrier.

### Brief description of the drawings

Figure 1. Heart tissue from patient #3 after 300 mg/m² DOX. Evaluation by electron microscopy demonstrated an abundant presence of microvacuoles in the cardiomyocytes.
Figure 2. Heart tissue from patient #3 after 480 mg/m² DOX. In addition to the vacuolization, a loss of myofibrils is observed with electron microscopy.

### Examples

In this example, we evaluated the cardioprotective properties of monoHER given as a 10 minute i.v infusion at a dose of 1500 mg/m² in patients with metastatic cancer treated with DOX. For the early detection of DOX-induced cardiotoxicity endomyocardial biopies were taken, because of its high sensitivity and high specificity (18).

### PATIENTS AND METHODS

### Patient selection

Patients with metastatic solid tumors were entered when they received a DOX-based chemotherapy regimen with a dosage of DOX ≥ 50 mg/m²/ cycle and an infusion duration ≤ 1 hour. Patients had a WHO performance status of ≤ 2 and a life expectancy of more than 3 months. They also had adequate organ functions as defined by a creatinine ≤ 140 µmol/l or a creatinine clearance ≥ 60 ml/min, serum bilirubin ≤ 26 µmol/l, alkaline phosphatase, ASAT and ALAT ≤ 2.5x the upper limit of normal (ULN), unless related to liver metastases, in which case ≤ 5x ULN was allowed. The left ventricular ejection fraction (LVEF) was > 50%. Patients were excluded if they had received prior anthracyclines, had prior or actual cardiovascular disease or had prior radiotherapy to the mediastinum.

All patients gave written informed consent and the protocol was approved by the medical ethical review committee of the VU University Medical Center (VUMC). Patients were enrolled between September 2003 and March 2006. Treatment

7-Monohydroxyethylrutoside (monoHER) was provided by Novartis Consumer Health (Nyon, Switzerland). The drug was formulated by the Department of Pharmacy, VUMC, Amsterdam and dissolved as described before (17). Formulated doxorubicin (Doxorubicin hydrochloride, 2 mg/ml) was obtained from Pharmachemie B.V. (Haarlem, the Netherlands).

MonoHER was administered as a 10 min intravenous infusion at a dose of 1500 mg/m² 60 min before every DOX administration. If cardiotoxicity would be observed in the first three evaluable patients, administration according to this dosing scheme would be changed and the following patients would receive DOX infusion either immediately after monoHER (because in plasma and heart Cₘₐₓ of monoHER is obtained immediately after the end of infusion (17, 19)) or with an interval of 2 hours (to give monoHER the opportunity to convert into an active metabolite, if any).

### Patient evaluation

Before starting the study patients were evaluated by a full blood count, serum biochemistry including liver function tests, lactate dehydrogenase (LDH) and cholesterol. Risk factors for cardiovascular disease were also evaluated. An LVEF and an ECG were performed before entry into the study.

Every subsequent administration of monoHER and DOX was preceded by a full blood count, liver enzymes, serum creatinine and a routine 12-lead ECG. A complete blood count was also done 10 days after the chemotherapy.

After a cumulative dose of 300 mg/m² DOX an endomyocardial biopsy was performed and measurement of the LVEF was repeated. The latter was also done at least 3 weeks after the last dose of DOX and a biopsy was repeated if possible. For logistic reasons, the biopsy of patient #8 was done after a cumulative dose of 375 mg/m² DOX.

### Endomyocardial biopsy

During a left heart catheterization, a 104 cm, 7 french biopsy forceps was used to obtain tissue from the left ventricle. Three to four specimens 0.5 to 1 mm in diameter were obtained. The specimens were fixed in 4% buffered formaldehyde and prepared for electron microscopy.

### Histological analysis and biopsy scores

After fixation in 4% buffered formaldehyde, the heart tissue was post fixed in 1% osmium tetroxide. The tissue was then dehydrated through a graded series of ethanol solutions of 70-95% and embedded in JB-4 Plus resin. Thereafter, 0.5-3.0 µm thick sections were cut with a glass knife. These semithin sections were processed for electron microscopy. Cardiomyocytes with more than 2 microvacuoles, macrovacuoles and/or loss of myofibrils were counted as deviant. The morphological grade determined from the specimens examined by electron microscopy was scored on a 6-point scale previously described by Billingham and Bristow (20, 21): in grade 0 cells are normal; in grade 1, 1.5, 2 and 2.5 deviant cells are < 5%, 5-15%, 16 - 25 % and 26 - 35%, respectively; in grade 3 cell damage is present in more than 35% of cells.

### Off-study criteria

Patients went off study in case of progressive disease, a serious cardiac event, or other events that precluded further treatment. Criteria described by Shapiro et al. (22) were used for diagnosis of cardiac events. Episodes of cardiac dysfunction were characterized according to the New York Heart Association functional classification (23).

### Assessment of tumor response

Assessment of tumor response was done every 2-3 cycles by CT-scan, using standard ECOG criteria (24).

### Statistical analysis

This trial was an open-labelled, controlled study. We compared the data of our patients with those of 14 patients from the study of Torti et al. (25) treated with a cumulative dose of DOX between 200-300 mg/m² alone using the Chi-Square test. Our hypothesis was that adding monoHER to DOX would eliminate its cardiotoxicity up to a cumulative dose of at least 300 mg/m², which was the upper limit of the dose interval of DOX from Torti's patients. In order to achieve a power of 80%, 11 patients would be required. This sample size was obtained when applying the Chi-Square test with significance level 0.05 and assuming a response rate (i.e. no cardiac damage) in the experimental arm of 80%. If at least 5 patients show DOX-induced damage, statistical significance cannot be achieved anymore and the study should be stopped.

### RESULTS

Eight patients meeting the inclusion criteria were enrolled (Table 1).

During the analysis of risk factors, it appeared that patient no. 6 was treated for hyperhomocysteinemia. Two other patients had an elevated body mass index of 29.1 (#1), indicating overweight, and 30.1(#7), indicating obesity, respectively. Patient no. 2 had hyperlipidemia with an elevated concentration of low-density lipoproteins (LDL) of 6.3 mmol/l (normal values ≤ 5.0). None of the other patients had risk factors for cardiovascular disease. All patients were classified as NYHA class I and had a performance status (WHO) between 0-2. Five patients received a cumulative dose ≥ 300 mg/m2 DOX and underwent a biopsy. In the other three patients DOX was discontinued before this dose, due to progressive disease.

### Treatment with DOX preceded by monoHER

During monoHER infusion two patients reported adverse events. One patient described a sensation of fullness in his stomach which developed during infusion of monoHER and which disappeared soon after the end of the infusion. The other patient experienced itching in the skin of the neck, starting during the infusion of monoHER and disappearing rapidly after the infusion. Both patients experienced the events during each cycle. A causal relationship cannot be excluded. The other 6 patients did not experience adverse events. None of the patients had a delay in receiving subsequent cycles of chemotherapy. As expected, all patients developed leucopenia due to chemotherapy that recovered before the start of the next cycle. No disturbances of liver enzymes and serum creatinine were noted.

### Biopsy scores

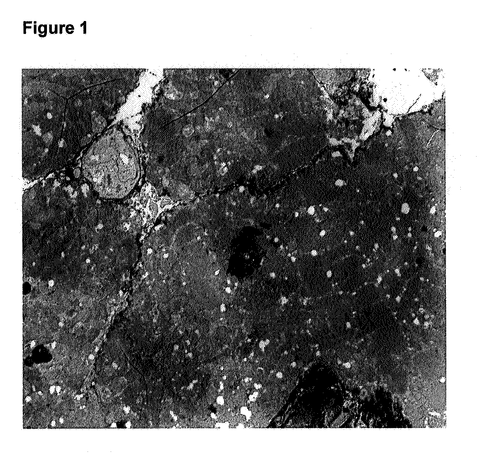

The endomyocardial biopsy scores are shown in Table 2. After 300 mg/m² the first three evaluable patients showed abnormalities consistent with DOX-induced cardiotoxicity i.e. the presence of microvacuoles dominated as is shown in Figure 1. In patient #3 and #4 microvacuoles were present in 52 and 20% of the cardiomyocytes, respectively. This was also observed in patient #6, where 33% of the cardiomyocytes had > 2 small vacuoles per cardiomyocyte. Because each of the first 3 evaluable patients showed DOX-induced damage, it was decided to stop this dosing scheme. The time-interval between monoHER and DOX administration was changed and patient #7 received DOX immediately after monoHER. In the biopsy of this patient microvacuolization was observed in 60% of the cardiomyocytes. After this result again the interval was changed and patient #8 received DOX 2 h after monoHER administration. In this patient also microvacuolization was detected in 49% of the cardiomyocytes. After these results, the study had to be considered negative, because no cardioprotective effect of monoHER was observed in the five patients.

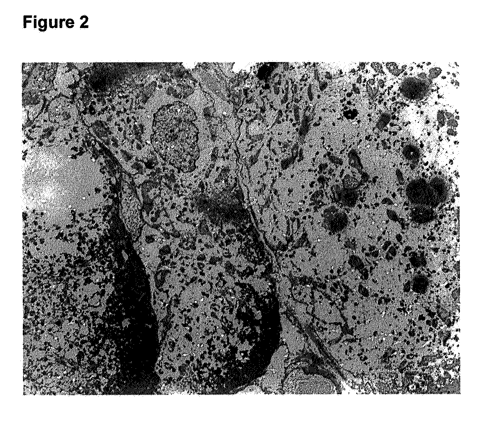

Second biopsies were also taken after 450-480 mg/m² of DOX in two patients (Table 3). The score of patient #4 had increased from 2.0 to 2.5, whereas the score of patient #3 remained 3. Striking was the increase in loss of myofibrils in the cardiomyocytes in addition to the microvacuoles observed after 300 mg/m² (Fig 2).

Ten months after his last cycle of DOX, patient #4 underwent a third biopsy. This time the cardiomyocytes showed recuperation of myofibrils, whereas the number of abnormal cardiac cells (microvacuolization) was less than that in the first two biopsies (score 2).

No complications occurred during the 7 biopsy procedures in the first four patients. However, the last patient (#8) developed a small pericardial effusion after the biopsy. This was preceded by chest pain and transient supraventricular rhythm disturbances. These sequelae disappeared within a few days and after a week she recovered and received the sixth cycle of DOX.

### Monitoring and evaluation of cardiac function

In all patients the ECG remained unchanged during therapy and no cardiac dysfunction occurred.

The LVEF score of the 5 patients who received at least 300 mg/m² DOX are shown in Table 2. All five patients started with an LVEF > 50%. After 300-375 mg/m² DOX, the LVEF decreased in 4/5 patients. This decrease was not related with the biopsy score or the time interval between monoHER and DOX infusions. Unfortunately, no information on the LVEF was available for patient #6.

Two patients received higher cumulative doses of DOX (#3 and #4). After 480 mg/m2 DOX, patient #3 had a decrease in LVEF to 53%, which is a decline of 25% compared to the ejection fraction before start of the study (71%). The patient had no symptoms of cardiac failure and even practiced fitness every day. Ten months after his last cycle of chemotherapy the LVEF remained stable and he remained without cardiac symptoms.

The LVEF of patient #4 showed an initial drop from 72% before starting chemotherapy to 60% after 300 mg/m2 DOX (decline of>15%). This value remained stable up to 450 mg/m² of DOX and at 10 month follow-up, the patient still had an excellent physical condition.

### Evaluation of tumor response

Response to the chemotherapy was remarkable in the 4 patients with metastatic soft tissue sarcoma (STS). Three of them developed a partial remission as observed on the CT scan (Table 1). In two of these patients (#4 and #7) partial remission was maintained up to the present, i.e., 30 and 16 months after the start of chemotherapy, respectively. The other patient (#3) had progressive disease after a partial remission of nine months duration. The fourth patient (#8) achieved stable disease for at least 7 months, while continuing therapy with DOX up to a cumulative dose of 495 mg/m².

### DISCUSSION

Based on the promising results with monoHER observed in preclinical experiments, we performed the present phase II study in patients with metastatic cancer. The cardioprotective effect of monoHER on DOX-induced cardiotoxicity was evaluated by endomyocardial biopsy. However, the results indicated that the preclinical observations were not translated into protection against DOX-induced heart damage in humans.

The golden standard for early detection of DOX-induced cardiotoxicity is the endomyocardial biopsy, because of its high sensitivity and high specificity (25). Currently, the most common method used to detect DOX-induced cardiac damage is the evaluation of the LVEF, but usually at later stages (26, 27). For detection of cardiotoxicity at an earlier stage, the use of biochemical markers such as atrial and brain natriuretic peptides, endothelin-1 as well as cardiac troponin-T and -I have been investigated (26, 28, 29). However, large-scale studies addressing whether these biomarkers following DOX treatment will be predictive for the development of late onset heart failure, are lacking.

Therefore, despite its invasiveness, we chose the endomyocardial biopsy for this evaluation.

Data of Billingham et al. (20) showed that anthracycline-induced myocardial damage occurred in nearly all patients treated with cumulative DOX doses of 240 mg/m². Therefore we could expect detectable heart damage after a cumulative dose of 300 mg/m², which would allow the registration of protection by monoHER. However, all 5 patients undergoing the biopsy procedure after a median cumulative dose of 300 mg/m2 showed DOX-induced cardiac damage with a mean biopsy score of 2.7 according to Billingham (20). This score was independent of the time interval between monoHER and DOX infusion and much higher than the biopsy score of 1.4, which is expected after a cumulative dose of 300 mg/m² DOX according to the linear regression analysis of Torti et al. (25). In our patients mainly one of the three morphological changes, i.e. small vacuoles of varying size were observed after 300 mg/m² DOX, whereas in Torti's study also partial or total myofibrillar loss was observed (25). However, in Torti's study it is not clear after which dose this occurred. In addition to microvacuolization, loss of myofibrils was demonstrated in two patients after higher cumulative doses of DOX. This suggests that the occurrence of microvacoles as such is a marker of cytotoxicity of DOX treatment. Billingham et al. (30) described that these microvacuoles, detected by electron microscopy, appear early as a swelling of the sarcoplasmic reticulum, which eventually coalesce to form large spaces in the cytoplasm (macrovacuoles). When, or if microvacuoles always become macrovacuoles is not clear from literature.

Thus, instead of protection, our patients had higher biopsy scores than the historical controls. However, it should be noted that the data of Torti et al. were obtained after a cumulative dose of 200-300 mg/m² with biopsies from the right ventricle (25), whereas biopsies from our patients were obtained after a cumulative dose of 300 mg/m² from the left ventricle. Previously, it was shown that ultrastructural abnormalities of myocytes were more pronounced in biopsy specimens from the left ventricle than those from the right ventricle (31). In addition to this, there is a considerable variability in patient sensitivity to the cardiotoxic effects of DOX (32, 33). This data may explain a possible overestimation of the toxic effect of DOX on the heart tissue in our patients. Although heart failure is directly related to the degree of myocyte damage (34), none of our patients developed heart failure, although the LVEF dropped by 16% in two patients, whereas it decreased in two other patients by only 4-6% (in comparison to the initial LVEF).

In the third biopsy procedure of patient #4, the score of the cardiac tissue had improved. A recuperation of myofibrils in the cardiomyocytes was observed and the microvacuolization was less. These findings are in agreement with a previous study reporting that some improvement of the histological damage may occur (35). This is in contrast with other results, which indicated that DOX-induced cardiotoxity is an ongoing progressive process (20).

The contrasting effects of monoHER found in animal and human studies may be attributed to differences in metabolism between the species. Therefore, patient #8 was treated with a 2 hour interval. However, this interval change did not reduce DOX-induced cardiac damage. In addition, no metabolites of monoHER have been detected until now. On the other hand, it cannot be excluded that during scavenging of highly reactive species in humans, the antioxidant monoHER is converted into a reactive oxidation product, which like the oxidation product of quercetin maybe prone to form adducts with thiol groups from glutathione and proteins (36). Depletion of glutathione may in addition to the low antioxidant status of the cardiac myocyte (37) reduce cardioprotection, while monoHER-protein adducts may cause additional toxicity (38).

Another unexpected observation in our study was that 3 of the four patients with STS had objective remissions, while the fourth patient had stable disease. Normally, objective responses on DOX in STS patients without prior chemotherapy have been reported to be approximately 25% (39). Although our observation was done in a very limited number of patients, our result is much better than expected, because the chance of observing an objective response in 4 consecutive STS patients treated with DOX is 0.4%. Thus, monoHER enhances the antitumour activity of DOX in STS.

As a consequence of the above-mentioned aspects, there may be a dose-depending transition in the effect of monoHER i.e. a high dose (≥1500 mg/m²) for obtaining a potentiating effect of the antitumour effect for at least soft tissue sarcomas and a low dose (somewhere below 1500 mg/m²) for obtaining cardioprotection. These aspects have to be elucidated further in the near future. It may be concluded that monoHER at a dose of 1500 mg/m² did not protect against DOX-induced cardiotoxicity in patients with metastatic disease. Further preclinical and clinical investigations seem to be warranted to investigate the dose-depending transitional effect of monoHER.
1. Steinherz LJ, Steinherz PG, Tan CTC, et al: Cardiac toxicity 4 to 20 years after completing anthracycline therapy. JAMA 266: 1672-1677, 2001
2. Lipshultz SE, Lipsitz SR, Sallan SE, et al: Chronic progressive cardiac dysfunction years after doxorubicin therapy for childhood acute lymphoblastic leukemia. J Clin Oncol 23: 2629-2636, 2005
3. Wouters KA, Kremer LCM, Miller TL, et al: Protecting against anthracycline-induced myocardial damage: a review of the most promising strategies. B J Haematol, 2005
4. Minotti G, Menna P, Salvatorelli E, et al: Anthracyclines: Molecular advances and pharmacologic developments in antitumor activity and cardiotoxicity. Pharmacol Rev 56: 185-229, 2004
5. Seidman A, Hudis C, Pierri MK, et al: Cardiac dysfunction in the trastuzumab clinical trials experience. J Clin Oncol 20: 1215-1221, 2002
6. Hrdina R, Gersl V, Klimtova I, et al: Anthracycline-induced cardiotoxicity. Acta Medica 43: 75-82, 2000
7. Horenstein MS, Vander Heide RS, L'Ecuyer TJ: Molecular basis of anthracycline-induced cardiotoxicity and its prevention. Mol Gen Metabol 71:436-444, 2000
8. Xu MF, Tang PL, Oian ZM, et al: Effects by doxorubicin on the myocardium are mediated by oxygen free radicals. Life Sci 68: 889-901, 2001
9. Iarussi D, Indolfi P, Galderisi M, et al: Cardiac toxicity after anthracycline chemotherapy in childhood. Herz 25:676-688, 2000
10. Doroshow JH, Locker GY, Meyers CE: Enzymatic defenses of the mouse heart against reactive oxygen metabolites. J Clin Invest 65: 128-135, 1980
11. Cvetkovic RS, Scott L Jr: Dexrazoxane: a review of its use for cardioprotection during anthracycline chemotherapy. Drugs 65: 1005-1024,2005
12. Van Dalen EC, Caron HN, Dickinson HO, et al: Cardioprotective interventions for cancer patients receiving anthracyclines. The Cochrane database of Systematic Reviews, Issue 1, 2005
13. Van Acker SA, Boven E, Kuiper K et al: Monohydroxyethylrutoside, a dose-dependent cardioprotective agent, does not affect the antitumor activity of doxorubicin. Clin Cancer Res 3: 1747-1754, 1997
14. Van Acker FA, van Acker SA, Kramer K, et al: 7-monohydroxyethylrutoside protects against chronic doxorubicin-induced cardiotoxicity when administered only once per week. Clin Cancer Res 6: 1337-1341, 2000
15. Haenen GRMM, Jansen FP, Bast A: The antioxidant properties of five O- (beta-hydroxyethyl) rutosides of the flavonoid mixture Venoruton. Phlebology : S10-S17, 1993 (suppl 1)
16. Van Acker SABE, Towart R, Husken BCP, et al: The protective effect of Venoruton and its constituents on acute doxorubicin-induced cardiotoxicity. Phlebology: S31-S32, 1993 (suppl 1)
17. Willems AM, Bruynzeel AME, Kedde MA, et al: A phase I study of monohydroxyethylrutoside in healthy volunteers. Cancer Chemother Pharmacol 57:678-684, 2006
18. Meinardi MT, van der Graaf WT, van Veldhuisen DJ, et al: Detection of anthracycline-induced cardiotoxicity. Cancer Treat Rev 25: 237-247, 1999
19. Abou El Hassan MAI, Kedde MA, Zwiers UTH, et al : Bioavailability and pharmacokinetics of the cardioprotecting flavonoid 7-monohydroxyethylrutoside in mice. Cancer Chemother Pharmacol 52: 371-376, 2003
20. Billingham ME, Mason JW, Bristow MR, et al: Anthracycline cardiomyopathy monitored by morphologic changes. Cancer Treat Rep 62: 865-872, 1978
21. Bristow MR, Lopez MB, Mason JW, et al: Efficacy and cost of cardiac monitoring in patients receiving doxorubicin. Cancer 50: 32-41, 1982
22. Shapiro C, Ervin T, Welles L, et al: Phase II trial of high-dose liposome-encapsulated doxorubicin with granulocyte colony-stimulating factor in metastatic breast cancer. J Clin Oncol 17:1435-1441, 1999
23. Seidman A, Hudis Pierri MK, et al: Cardiac dysfunction in the trastuzumab clinical trials experience. J Clin Oncol 20: 1215-1221, 2002
24. Oken MM, Creech RH, Tormey DC, et al: Toxicity and response criteria of the Eastern Cooperative Oncology Group. Am J Clin Oncol 5: 649-655, 1982
25. Torti FM, Bristow MM, Lum BL, et al: Cardiotoxicity of epirubicin and doxorubicin: assessment by endomyocardial biopsy. Cancer Research 46:3722-3727, 1986
26. Kilickap S, Barista I, Akgul E, et al: cTnT can be a useful marker for early detection of anthracycline cardiotoxicity. Ann Oncol 16: 798-804, 2005
27. Villani F, Meazza R, Materazzo C: Non-invasive monitoring of cardiac hemodynamic parameters in doxorubicin-treated patients: comparison with echocardiography. Anticancer Res 26: 797-801, 2006
28. Suzuki T, Hayashi D, Yamazaki T, et al: Elevated B-type natriuretic peptide levels after anthracycline administration. Am Heart J 136: 362-363, 1998
29. Yamashita J, Ogawa M, Shirakusa T: Plasma endothelin-1 as a marker for doxorubicin cardiotoxicity. Int J Cancer 62: 542-547, 1995
30. Billingham ME, Bristow MR, Glatstein E, et al: Adriamycin cardiotoxicity: endomyocardial biopsy evidence of enhancement by irradiation. Am J Surg Pathol 1: 17-23, 1977
31. Mortensen SA, Olsen HS, Baandrup U: Chronic anthracycline cardiotoxicity: haemodynamic and histopathological manifestations suggesting a restrictive endomyocardial disease. Br Heart J 55: 274-282, 1986
32. Mason JW, Bristow MR, Billingham ME, et al: Invasive and noninvasive methods of assessing adriamycin cardiotoxic effects in man: superiority of histopathologic assessment using endomyocardial biopsy. Cancer Treat Rep 62: 857-864, 1978
33. Ferrans VJ: Overview of cardiac pathology in relation to anthracycline cardiotoxicity. Cancer Treat Rep 62: 955-961, 1978
34. Bristow MR, Billingham ME, Mason JW, et al: Clinical spectrum of anthracycline antibiotic cardiotoxicity. Cancer Treat Rep 62: 873-879, 1978
35. Mackay B, Ewer MS, Carrasco CH, et al: Assessment of anthracycline cardiomyopathy by endomyocardial biopsy. Ultrastructural Pathology 18: 203-211, 1994
36. Boots AW, Balk JM, Bast A, et al: The reversibility of glutathionyl-quercetin adducts spreads oxidized quercetin-induced toxicity. Biochem Biophys Res Commun 338: 923-929, 2005
37. Nowak D, Drzwoski J: Anthracycline-induced oxidative stress - its role in the development of cardiac damage. Cancer J 9: 296-303, 1996
38. Boots AW, Bast A, Haenen GRMM: No role of DT-diaphorase (NQO1) in the protection against oxidized quercetin. FEBS Lett 579: 677-682, 2005
39. Santoro A, Tursz T, Mouridsen H, et al: Doxorubicin versus CYVADIC versus doxorubicin plus ifosfamide in first-line treatment of advanced soft tissue sarcomas: a randomized study of the European organization for research and treatment of cancer soft tissue and bone sarcoma group. J Clin Oncol 13: 1537-1545, 1995

**Table 1. Patient characteristics.**

| Patient # | Age /Sex | Diagnosis | Total dose of dox (mg/m²) | Response on dox | Biopsy |
|---|---|---|---|---|---|
| 01 | 62 / F | Breast cancer | 100 | PD | N |
| 02 | 54 / F | Adrenal cortical cancer | 150 | PD | N |
| 03 | 25 / M | Malignant peripheral nerve sheet tumor | 480 | PR | Y |
| 04 | 64 / M | Malignant fibrous histiocytoma | 450 | PR | Y |
| 05 | 55 / F | Breast cancer | 100 | PD | N |
| 06 | 48 / F | Breast cancer | 300 | SD | Y |
| 07 | 45 / F | Malignant fibrous histiocytoma | 300 | PR | Y |
| 08 | 56 / F | Malignant fibrous histiocytoma | 375 | SD | Y |

| | | | | | |
|---|---|---|---|---|---|
| Age in years; sex F female, M male; Assessment of tumor response was done by using standard ECOG criteria (PD = progressive disease; PR = partial remission; SD = stabile disease); Y yes, N no. | | | | | |

**Table 2. Patients who received at least a cumulative dose (cum. dose) of 300 mg/m2 DOX and underwent an endomyocardial biopsy.**

| Patient # | Biopsy score cum.dose/Δt/grade | LVEF before dox | LVEF after ≥ 300 mg/m² |
|---|---|---|---|
| 03 | 300 / 60 / Grade 3 | 71% | 67% |
| 04 | 300 / 60 / Grade 2 | 72% | 60% |
| 06 | 300 / 60 / Grade 2.5 | 52% | ND |
| 07 | 300 / 10 / Grade 3 | 75% | 63% |
| 08 | 375 / 120 / Grade 3 | 65% | 63% |

| | | | |
|---|---|---|---|
| Δt = time between end of monoHER infusion and start of DOX infusion in min. The morphological grade was scored on a 6-point scale previously described by Billingham and Bristow; LVEF = left ventricular ejection fraction. | | | |

**Table 3. Two patients with more than one endomyocardial biopsy.**

| Patient # | Cum.dose / biopsy score / LVEF | Biopsy score / LVEF 10 months after last cycle |
|---|---|---|
| 03 | 480 / Grade 3 / 53% | ND / 51% |
| 04 | 450 / Grade 2.5 / 66% | Grade 2 / 62% |

| | | |
|---|---|---|
| The morphological grade was scored on a 6-point scale previously described by Billingham and Bristow; LVEF = left ventricular ejection fraction; ND = not done. | | |

## Claims

1. Use of a polyphenolic flavonoid according to formula 1 in the manufacture of a medicament for the treatment of an individual suffering from cancer, whereby said polyphenolic flavonoid acts synergistically with a chemotherapeutic drug; and whereby said individual is a human 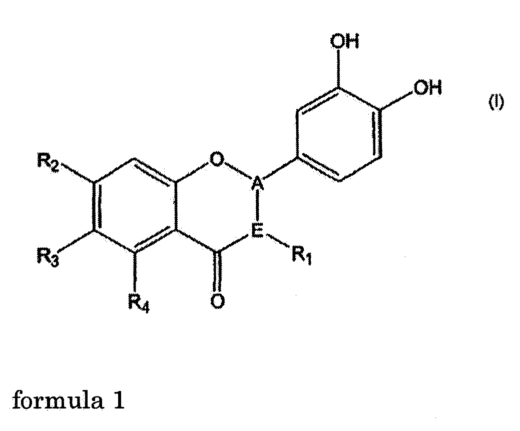 wherein: A and E form together a C-C or C=C bond,
R₁, R₂, R₃, and R₄ are each independently chosen from:
H;
OH;
(R')ₙ R" and/or O(R')ₙ R" wherein n=0-8 and
R' is independently chosen from CH₂ or O(CH₂)_{n'} wherein n'=1-4 and
R" when
n=0 is independently chosen from
H;
an aromatic group; a C₁-C₈ alkyl group; a sugar in mono, di or trimeric form or an analogue thereof;
NRR'''; N(R''')₂; N(R''')₃-halide;
halide;
COOR''';
CONHR'''; CON(R''')₂; or CON(R''')₃-halide wherein
R''' is independently chosen from H; an aromatic group; a C₁-C₈ alkyl group; or a sugar in mono, di or trimeric form or an analogue thereof;
and when
n=1-8 is independently chosen from
H;
an aromatic group; a C₁-C₈ alkyl group; a sugar in mono, di or trimeric form or an analogue thereof;
NHR'''; N(R''')₂; N(R''')₃-halide;
OH;
halide;
COOR''';
CONHR'''; CON(R''')₂; or CON(R''')₃-halide wherein
R''' is H; an aromatic group; a C₁-C₈ alkyl group; or a sugar in mono, di or trimeric form or an analogue thereof;
(R')ₙ OR" wherein n=1-8 and
R' is independently chosen from CH₂ or O(CH₂)_{n'} wherein n'=1-4 and
R" is independently chosen from
H;
an aromatic group; a C₁-C₈ alkyl group; a sugar in mono, di or trimeric form or an analogue thereof;
NHR'''; N(R''')₂; N(R''')₃-halide;
H;
halide;
COOR''';
CONHR'''; CON(R''')₂; or CON(R''')₃-halide wherein
R''' is H; an aromatic group; a C₁-C₈ alkyl group; or a sugar in mono, di or trimeric form or an analogue thereof;

2. Use according to claim 1, wherein said chemotherapeutic drug comprises an anthracycline.

3. Use according claim 2, wherein said anthracycline comprises doxorubicin.

4. Use according to any of claims 1-3, wherein said polyphenolic flavonoid of formula 1 is hydroxy-ethylated and/or hydroxy-methylated.

5. Use according to any one of claims 1-4, wherein the compound comprises monohydroxyethylrutoside.

6. Use according to any one of claims 1-5, with the proviso that said compound is not a compound wherein R₁= OH, R₂= OH, R₃= H and R₄= OH.

7. Use according to any one of claims 1-6, wherein said compound is not a naturally occurring polyphenolic flavonoid.

8. Use according to any of the previous claims, wherein said treatment of an individual suffering from cancer comprises treatment of an individual suffering from a soft tissue sarcoma.

9. Use according to claim 8, further comprising the treatment of an individual suffering from metastases of said sarcoma.

10. Use according to claim 8 or claim 9, wherein said sarcoma is selected from the group comprising alveolar soft part sarcoma, angiosarcoma, dermatofibrosarcoma, desmoid tumor, desmoplastic small round cell tumor, mesenchymoma, clear cell sarcoma, chondrosarcoma, fibrosarcoma, hemangiopericytoma, hemangiosarcoma, Kaposi's sarcoma, leiomyosarcoma, liposarcoma, lymphangiosarcoma, malignant fibrous histiocytoma, neurofibrosarcoma, rhabdomyosarcoma , synovial sarcoma, and peripheral nerve sheet tumor.

11. Use according to claim 10, wherein said sarcoma is selected from the group comprising fibrous histiocytoma and peripheral nerve sheet tumor.

12. Use according to any of the previous claims, whereby said polyphenolic flavonoid is administered to said individual by intravenous infusion at a dose of between 100 and 3000 mg/m².

13. Use according to any of the previous claims, wherein said polyphenolic flavonoid is administered prior to administering said chemotherapeutic drug.

14. Use according to claim 13, wherein said chemotherapeutic drug administration is started between 0 and 2 hours after the start of the administration of said polyphenolic flavonoid.

15. A method for the treatment of an individual suffering from cancer, comprising administering to said individual a medicament comprising a polyphenolic flavonoid and a medicament comprising a chemotherapeutic drug, whereby said flavonoid acts synergistically with said chemotherapeutic drug; and whereby said individual is a human.

16. Method according to claim 15, wherein said chemotherapeutic drug comprises an anthracycline.

17. Method according to claim 15, wherein said polyphenolic flavonoid is administered prior to administering said chemotherapeutic drug.

18. Use of a polyphenolic flavonoid for the manufacture of a medicament to enhance the chemotherapeutic effect of a chemotherapeutic drug in human.

19. A pharmaceutical composition, comprising a polyphenolic flavonoid and an acceptable carrier.
